# EUROPÄISCHE PATENTSCHRIFT

(11) **EP 2 254 498 B1**
(45) Veröffentlichungstag und Bekanntmachung des Hinweises auf die Patenterteilung: **23.09.2015**
(21) Anmeldenummer: 09713503.2
(22) Anmeldetag: 18.02.2009
(51) Int. Cl.: A61C 1/00, A61C 1/06, A61B 17/16, A61C 1/18

(54) **VORRICHTUNG ZUM BETREIBEN EINES ELEKTRISCH BETRIEBENEN MEDIZINISCHEN INSTRUMENTS**
DEVICE FOR OPERATING AN ELECTRICALLY OPERATED MEDICAL INSTRUMENT
DISPOSITIF DESTINÉ AU FONCTIONNEMENT D UN INSTRUMENT MÉDICAL À FONCTIONNEMENT ÉLECTRIQUE

(30) Priorität: 18.02.2008 DE 102008009623
(43) Veröffentlichungstag der Anmeldung: 01.12.2010
(73) Patentinhaber: Kaltenbach & Voigt GmbH, 88400 Biberach (DE)
(72) Erfinder: SAUTER, Johannes, 88416 Mittelbuch (DE); BÜRK, Richard, 88433Alberweiler (DE)
(74) Vertreter: Thun, Clemens
(86) Internationale Anmeldenummer: PCT/EP2009/001149
(87) Internationale Veröffentlichungsnummer: WO 2009/103509

(56) Entgegenhaltungen:
- EP-A- 0 549 910
- EP-A- 0 890 343
- EP-A- 1 084 683
- DE-A1-102004 014 667

## Beschreibung

Die vorliegende Erfindung betrifft eine Vorrichtung zum Betreiben eines medizinischen, insbesondere eines zahnmedizinischen Instruments, welches als Antriebseinheit einen Elektromotor aufweist, wobei die Vorrichtung das Instrument abhängig von einer der Vorrichtung zugeführten Druckluft ansteuert.

Vorrichtungen dieser Art sind bereits grundsätzlich bekannt und werden von der Anmelderin bspw. unter der Bezeichnung ELECTROtorque TLC oder ELECTROtorque Plus vertrieben. Sinn und Zweck dieser Vorrichtungen ist es, auch bei älteren zahnärztlichen Behandlungseinrichtungen, bei denen als Antriebsmedium für die verschiedenen Instrumente ausschließlich Druckluft zur Verfügung steht, die Möglichkeit zu eröffnen, Instrumente der neueren Generation einzusetzen, welche auf einem Elektroantrieb basieren. Derartige Elektromotor-Handstücke weisen gegenüber klassischen Turbinenhandstücken deutliche Vorteile hinsichtlich ihrer Betriebseigenschaften auf. So besteht beispielsweise die Möglichkeit, bei derartigen elektrischen Antrieben eine Einstellung der Drehzahl und des Drehmoments vorzunehmen.

EP 0 890 343 und DE 10 2004 014667 offenbaren auch vorrichtungen dieser Art.

Die bekannten Vorrichtungein dienen dementsprechend dazu, die von der zahnärztlichen Behandlungseinheit zur Verfügung gestellte Druckluft, welche bspw. mit Hilfe eines sog. Fußanlassers eingestellt werden kann, in ein der Höhe Luftdrucks entsprechendes Ansteuersignal für den Elektroantrieb umzusetzen. Üblicherweise wird die Druckluft in einen variablen Motorstrom umgesetzt, um die Drehzahl für den Motor einzustellen.

Da derartige Vorrichtung zur Umsetzung der Druckluft in ein Ansteuersignal für das elektrisch betriebene Instrument an unterschiedlichsten Behandlungseinheiten zum Einsatz kommen sollen, muss Sorge dafür getragen werden, dass diese die verschiedenen Versorgungsluftdrücke der Behandlungseinheiten, welche sich in verschiedenen Praxen stark bezüglich des maximal zur Verfügung gestellten Drucks unterscheiden können, effektiv nutzen bzw. umsetzen können. Bei den oben angesprochenen bekannten Geräten war deshalb vor dem erstmaligen Gebrauch jeweils ein Abgleichvorgang erforderlich, bei dem die maximale Motordrehzahl auf den maximalen Systemdruck der Behandlungseinheit abgeglichen werden musste. Im Rahmen dieser Prozedur mussten mehrere einzelne Schritte durchgeführt werden, um letztendlich den Abgleichvorgang abzuschließen, was eine gewisse Zeit kostete und aufwendig war.

Der vorliegenden Erfindung liegt deshalb die Aufgabe zugrunde, bei derartigen Vorrichtungen, die zur Umsetzung eines Versorgungsluftdrucks in ein Ansteuersignal für ein elektrisch betriebenes zahnmedizinisches Instrument vorgesehen sind, den Abgleichvorgang komfortabler zu gestalten.

Die Aufgabe wird durch die in den unabhängigen Ansprüchen angegebene Erfindung gelöst. Vorteilhafte Weiterbildungen der Erfindung sind Gegenstand der abhängigen Ansprüche.

Die erfindungsgemäße Lösung beruht auf der Idee, den erforderlichen Abgleichvorgang automatisch bzw. im Hintergrund ablaufen zu lassen, so dass es also nicht mehr erforderlich ist, dass der Zahnarzt oder ein das Gerät verwendender Dentaltechniker bei Inbetriebnahme einen komplizierten Abgleichvorgang ausführen muss. Dies wird erfindungsgemäß dadurch erzielt, dass die Kennlinie, gemäß der der Druck der zugeführten Druckluft in das Ansteuersignal für das medizinische Instrument umgesetzt wird, mit Hilfe bestimmter Bezugspunkte definiert wird, wobei während des Betriebs der Vorrichtung kontinuierlich der maximale Druck der zugeführten Druckluft bestimmt und auf Basis dieses ermittelten Maximaldrucks ein Bezugspunkt für die Kennlinie ermittelt wird.

Erfindungsgemäß wird dementsprechend eine Vorrichtung zum Betreiben bzw. Ansteuern eines elektrisch betriebenen medizinisches Instruments, insbesondere eines zahnärztlichen Behandlungsinstruments vorgeschlagen, wobei das medizinische Instrument mit der Vorrichtung zu verbinden ist und wobei die Vorrichtung einen Anschluss für eine Druckluftleitung sowie eine Steuereinheit aufweist, welche dazu ausgebildet ist, den Druck der zugeführten Druckluft entsprechend einer Kennlinie in ein Ansteuersignal für das medizinische Instrument umzusetzen, und wobei erfindungsgemäß die Steuereinheit während des Betriebs kontinuierlich den maximalen Druck der Druckluft ermittelt und auf Basis dieses Maximaldrucks einen Bezugspunkt der Kennlinie bestimmt.

Gemäß einem weiteren Aspekt der vorliegenden Erfindung wird ein Verfahren zum Betreiben bzw. Ansteuern eines elektrisch betriebenen medizinischen Instruments, insbesondere eines zahnärztlichen Behandlungsinstruments vorgeschlagen, wobei die Höhe des Drucks einer eingangsseitigen Druckluft entsprechend einer Kennlinie in ein Ansteuersignal für das medizinische Instrument umgesetzt und anschließend das Ansteuersignal dem medizinischen Instrument zugeführt wird. Erfindungsgemäß ist hierbei vorgesehen, dass während des Betriebs kontinuierlich der maximale Druck der Druckluft ermittelt und auf Basis dieses Maximaldrucks ein Bezugspunkt der Kennlinie bestimmt wird.

Dadurch, dass der Bezugspunkt für die Kennlinie auf Basis des während des Betriebs kontinuierlich ermittelten Maximaldrucks bestimmt wird, kann der erforderliche Abgleich der Vorrichtung selbstständig im Hintergrund ablaufen, ohne dass ein Benutzer der Vorrichtung zur Inbetriebnahme einen speziellen Abgleichvorgang durchführen müsste. Während es beim Stand der Technik erforderlich war, dass Gerät zunächst in einen speziellen Abgleichmodus zu versetzen und dann entsprechende Betätigungen - bspw. des Fußanlassers der Behandlungseinheit - durchzuführen, wird nunmehr die Kennlinie automatisch an die aktuellen Druckbedingungen des Systems angepasst. Letztendlich ist zum Erstellen der optimalen Kennlinie lediglich erforderlich, dass der Benutzer der Vorrichtung bei der ersten Inbetriebnahme bspw. den Fußanlasser voll betätigt, was ohnehin erforderlich ist, um die Betriebsbereitschaft des Systems kurz zu überprüfen. Weitere Maßnahmen sind allerdings nicht mehr erforderlich, wodurch also der Bedienungskomfort der Vorrichtung deutlich erhöht wird.

Vorzugsweise ist in der Vorrichtung ein vorgegebener Mindestdruck gespeichert, der einen weiteren Bezugspunkt der Kennlinie definiert. Dieser Mindestdruck entspricht dem Druckwert, bei dem der Motor des medizinischen Instruments gestartet werden soll. Durch diese beiden Bezugspunkte wird dann die Kennlinie zur Umsetzung des Luftdrucks in das Ansteuersignal definiert, wobei insbesondere vorgesehen ist, dass die Kennlinie zwischen beiden Bezugspunkten linear verläuft. Durch lineare Erhöhung des Luftdrucks kann also bspw. die Drehzahl des Motors, die Leistung oder aber auch die Abtragsleistung bzw. das ausgeübte Drehmoment eines zahnärztlichen Behandlungswerkzeugs linear eingestellt werden. Alternativ hierzu könnte allerdings die Kennlinie zwischen den beiden Bezugspunkten auch entsprechend einer anderen mathematischen Funktion verlaufen. So wäre möglicherweise auch die Verwendung einer Exponentialfunktion denkbar, um eine feinere Drehzahlregelung bei niedrigen Drehzahlen zu ermöglichen.

Alternativ zu der zuvor beschriebenen Vorgehensweise, nämlich einen weiteren Bezugspunkt der Kennlinie auf Basis eines gespeicherten und vorgegebenen Mindestdrucks zu definieren, kann ferner auch vorgesehen sein, dass auch dieser zweite Bezugspunkt während des Betriebs automatisch angepasst wird. Insbesondere kann vorgesehen sein, dass der weitere Bezugspunkt der Kennlinie auf Basis eines von der Steuereinheit während des Betriebs ermittelten Minimaldrucks festgelegt wird. Vorzugsweise ermittelt hierbei die Steuereinheit während des Betriebs einen neuen Minimaldruck immer dann, wenn nach einem Druckabfall unterhalb des aktuell festgelegten Minimaldrucks der Druck innerhalb eines vorgegebenen Zeitraums im Wesentlichen konstant bleibt. Dieses Kriterium kann beispielsweise dann als verfüllt angesehen werden, wenn die Druckvarianz innerhalb eines Zeitraums von 0,5 Sekunden unterhalb 0,1 bar liegt. Der Vorteil dieser automatischen Anpassung an den minimalen Arbeitsdruck besteht darin, dass jederzeit sichergestellt ist, dass der zur Verfügung stehende Drehzahlbereich des Behandlungsinstruments voll genutzt werden kann.

Gemäß einer vorteilhaften Weiterbildung der Erfindung weist die erfindungsgemäße Vorrichtung ferner einen nicht-flüchtigen Speicher zur Speicherung des ermittelten Maximaldrucks sowie des ggf. ermittelten Minimaldrucks auf. Auch bei einer vorübergehenden Deaktivierung der Vorrichtung steht dementsprechend bei Neustart unmittelbar die korrekte Kennlinie zur Verfügung. Hierbei kann selbstverständlich auch vorgesehen sein, dass die Kennlinie manuell zurückgesetzt werden kann, um einen Neuabgleich zu ermöglichen.

Des Weiteren kann in vorteilhafter Weise vorgesehen sein, dass bei jedem Motorstart der max. Abgleichdruck leicht angepasst, insbesondere reduziert wird, um das System automatisch an geänderte Druckbedingungen anzupassen. Ist ferner auch eine Speicherung des ermittelten Minimaldrucks vorgesehen, so wird der gespeicherte Wert im Falle eines Starts zunächst leicht angehoben.

In besonders vorteilhafter Weise stellt die Vorrichtung gleichzeitig auch eine Stromversorgungsquelle für das zu betreibende medizinische Instrument dar. In diesem Fall kann dann das Ansteuersignal für das medizinische Instrument in Form eines variablen Versorgungsstroms für den Elektromotor zur Verfügung gestellt werden. Hier ist dann also zur Versorgung des Instruments lediglich eine einzige Verbindungsleitung zwischen Vorrichtung und dem Instrument erforderlich.

Letztendlich wird durch die erfindungsgemäße Lösung der Komfort bei der Benutzung derartiger Vorrichtungen, mit deren Hilfe ein Druckluftsignal in ein Ansteuersignal für ein elektrisch betriebenes medizinisches Instrument umgesetzt wird, deutlich erhöht.

Nachfolgend soll die Erfindung anhand der beiliegenden Zeichnung näher erläutert werden. Es zeigen:
- Fig. 1: eine schematische Darstellung eines zahnärztlichen Behandlungsplatzes zur Verdeutlichung der Funktionsweise der erfindungsgemäßen Vorrichtung;
- Fig. 2: die Ansicht einer Ausführungsform der erfindungsgemäßen Vorrichtung;
- Fig. 3 und 4: Grafiken zur Darstellung der erfindungsgemäßen Vorgehensweise zur Erstellung der Kennlinie für die Ansteuerung des medizinisches Instruments; und
- Fig. 5 und 6: Grafiken zur Darstellung einer vorteilhaften Weiterbildung des Verfahrens, bei der auch der untere Bezugspunkt der Kennlinie automatisch angepasst wird.

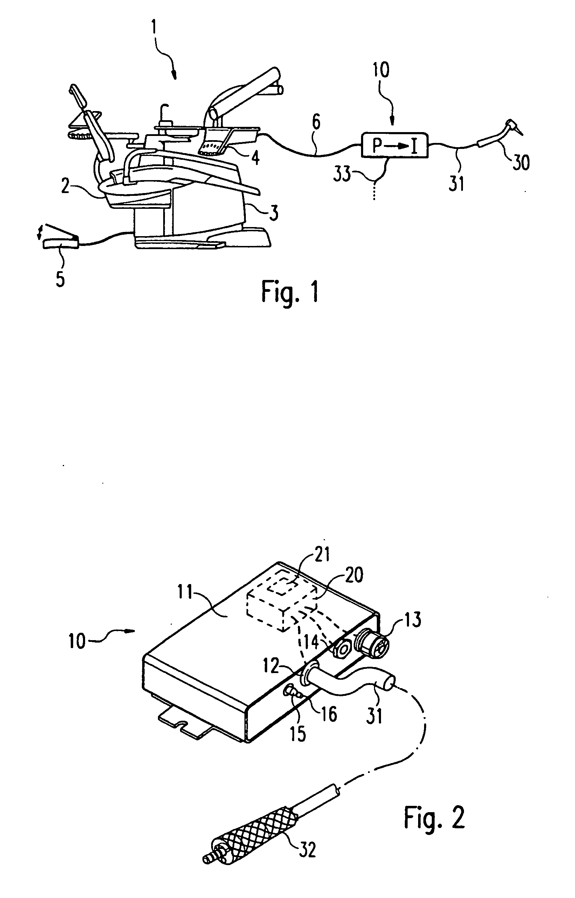

Anhand von Fig. 1 soll zunächst die grundsätzliche Funktionsweise der erfindungsgemäßen Vorrichtung erläutert werden. Hierzu ist ein allgemein mit dem Bezugszeichen 1 versehener zahnärztlicher Behandlungsplatz dargestellt, an dem die Vorrichtung 10 zum Einsatz kommen soll.

Bei dem dargestellten Behandlungsplatz handelt es sich um einen klassischen zahnmedizinischen Arbeitsplatz 1 mit einem Behandlungsstuhl 2, einer zentralen Steuer- und Versorgungseinheit 3 sowie einer daran angeordneten Instrumentenablage 4, welche zur Halterung mehrerer unterschiedlicher zahnärztlicher Behandlungsinstrumente genutzt werden kann. Bei diesen Behandlungsinstrumenten kann es sich bspw. um Zahnsteinentfernungsgeräte oder zahnmedizinische Bohrhandstücke handeln, welche üblicherweise während des Betriebs in ihrer Drehzahl bzw. Leistung eingestellt werden können. Die Ansteuerung erfolgt dabei in klassischer Weise über einen sog. Fußanlasser 5, dessen Pedal durch den Zahnarzt während der Behandlung durch den Fuß betätigt werden kann, um die Leistung zu verändern.

Grundsätzlich gesehen können zahnärztliche Handstücke mit unterschiedlichen Antrieben ausgestaltet sein. Dabei werden insbesondere Luft- bzw. Turbinenantriebe oder elektrische Antriebe eingesetzt. Eine Turbine zeichnet sich durch ihre kompakte Bauweise aus, andererseits ist die Betriebssicherheit im Vergleich zu einem Elektromotor üblicherweise geringer. Ferner bestehen im Vergleich zu einem Elektromotor nur begrenzte Möglichkeiten, die zu übertragende Leistung genau zu steuern.

Ältere zahnmedizinische Behandlungseinheiten nutzten trotz allem bislang in erster Linie Luft- bzw. Turbinenantriebe. Bei Betätigung des Fußanlassers 5 wurde dementsprechend von der zentralen Steuer- und Versorgungseinheit 3 eine Druckluft zur Verfügung gestellt, deren Druck in Abhängigkeit von der Einstellung des Fußpedals 5 variierte. An den Druckluft-Versorgungsschlauch konnten dann pneumatisch betriebene Handstücke oder Turbinenhandstücke angeschlossen werden. Die Möglichkeit allerdings, auch medizinische Instrumente mit Elektroantrieben einzusetzen, bestand bei derartigen Behandlungseinheiten nicht.

Um deshalb den Funktionsumfang solcher älterer Behandlungseinheiten zu erweitern und insbesondere die Möglichkeit zu öffnen, auch elektrobetriebene Instrumente einzusetzen, wurde die Ansteuervorrichtung 10 entwickelt, deren Aufgabe darin besteht, die von der Behandlungseinheit 1 zur Verfügung gestellte Druckluft in ein geeignetes Ansteuersignal für Instrumente mit Elektroantrieb umzusetzen. Wie Fig. 1 zeigt wird hierzu eingangsseitig die Vorrichtung 10 mit dem Druckluft-Versorgungsschlauch 6 der Behandlungseinheit 1 verbunden, wobei der Luftdruck der über die Leitung 6 zur Verfügung gestellt wird, in Abhängigkeit von der Betätigung des Fußanlassers 5 variiert. Derartige Änderungen des Luftdrucks werden von einer später noch näher beschriebenen internen Steuereinheit der Vorrichtung 10 registriert und in ein Ansteuersignal umgesetzt, welches über eine ausgangsseitige Leitung 31 dem medizinischen Instrument 30, bei dem es sich im dargestellten Ausführungsbeispiel um ein zahnmedizinisches Bohrhandstück handelt, zur Verfügung gestellt. Vereinfacht gesagt setzt also die Vorrichtung 10 den von der Behandlungseinheit 1 zur Verfügung gestellten Luftdruck in entsprechende Steuersignale zur Ansteuerung des Instruments 30 um. Auf diesem Wege können mit einem verhältnismäßig geringen Aufwand auch ältere zahnärztliche Behandlungseinheiten, welche lediglich Druckluft als Versorgungsmedium zur Verfügung stellen, zum Betreiben elektrobetriebener Instrumente der neueren Generation genutzt werden. Insbesondere kann auch der Fußanlasser der Behandlungseinheit genutzt werden.

Anzumerken ist, dass über die Leitung 6 nicht nur Druckluft an die Vorrichtung 10 übermittelt wird, sondern - wie nachfolgend noch näher erläutert - auch noch weitere Medien geleitet werden können, die während einer zahnärztlichen Behandlung genutzt werden. So wäre insbesondere auch die Übermittlung von Luft und/oder Wasser denkbar. Ferner wird die Vorrichtung 10 über eine separate, also von der Einheit 1 unabhängige Netzteilverbindung 33 mit Strom versorgt.

Fig. 2 zeigt nunmehr anhand einer vergrößerten Darstellung ein konkretes Ausführungsbeispiel einer erfindungsgemäßen Vorrichtung 10. Diese weist an der Vorderseite ihres Gehäuses 11 verschiedene Anschlüsse auf, welche nachfolgend näher erläutert werden sollen.

Ein erster Anschluss 12 dient hierbei zum Anschluss der Verbindungsleitung 31 zu dem medizinischen Instrument. Diese Leitung wird üblicherweise als Motorschlauch bezeichnet, da über sie nicht nur einzelne Ansteuersignale übertragen werden sondern auch die zum Betrieb des Instruments erforderlichen Medien zur Verfügung gestellt werden. So besteht bspw. die Möglichkeit, über diesen Motorschlauch 31 nicht nur Strom zum Betreiben des Elektromotors 32 zur Verfügung zu stellen. Stattdessen können ergänzend auch weitere in der Zahnmedizin üblicherweise verwendete Medien wie Luft und/oder Wasser übermittelt werden. Derartige Medien dienen insbesondere auch dazu, während der Behandlung die zu bearbeitende Stelle zu reinigen bzw. zu spülen. Der Anschluss 12 stellt dementsprechend neben Strom ggf. auch Luft und/oder Wasser zur Verfügung.

Bei dem zweiten Anschluss 13 handelt es sich um den Anschluss, über den die Verbindung zu der zahnärztlichen Behandlungseinheit 1 hergestellt wird. An diesen weiteren Anschluss 13 wird also der von der Einheit 1 wegführende Druckluft-Versorgungsschlauch 6 angeschlossen. Auch dieser Anschluss 13 dient - wie oben erwähnt - nicht nur zur Übermittlung der für die Ansteuerung des medizinischen Instruments als Eingangssignal benötigten Druckluft. Stattdessen können über diesen Anschluss 13 auch die weiteren Medien wie Luft und/oder Wasser von der Einheit 1 übermittelt werden. Vorzugsweise ist dementsprechend der Anschluss 13 als sog. Vier-Loch-Anschluss ausgebildet.

Bei dem dritten Anschluss 14 handelt es sich um einen Netzteil-Anschluss zur Stromversorgung. Die Stromversorgung dient hierbei nicht nur zum Betrieb der Vorrichtung 10 sondern auch als Stromversorgung für das medizinische Instrument 30 bzw. für den Elektromotor 32. In diesem Sinne dient die Vorrichtung 10 nicht nur zur Ansteuerung des Instruments 30 sondern auch als Stromversorgungseinheit, was nachfolgend noch näher erläutert wird.

Die Bezugszeichen 15 und 16 schließlich beziehen sich auf eine LED-Anzeige, welche den Bereitschaftszustand der Vorrichtung 10 anzeigt, sowie auf einen Taster, mit dem - wie nachfolgend erläutert - das Ergebnis des Druckluftabgleichs zurückgesetzt werden kann. Aufgrund der erfindungsgemäßen Vorgehensweise zur Durchführung des Abgleichs stellt dieser Taster 16 letztendlich das einzige Bedienelement der erfindungsgemäßen Vorrichtung 10 dar.

Weitere Komponenten der Vorrichtung sind eine interne Steuereinheit 20 sowie ein dieser Steuereinheit 20 zugeordneter Speicher 21. Die Steuereinheit steht dabei mit den Anschlüssen 12, 13, 14 in Verbindung, um die Höhe des über den Anschluss 13 erhaltenen Luftdrucks in ein entsprechendes Steuersignal umzusetzen, welches über den Anschluss 12 an den Versorgungsschlauch bzw. das medizinische Instrument 30 abgegeben wird.

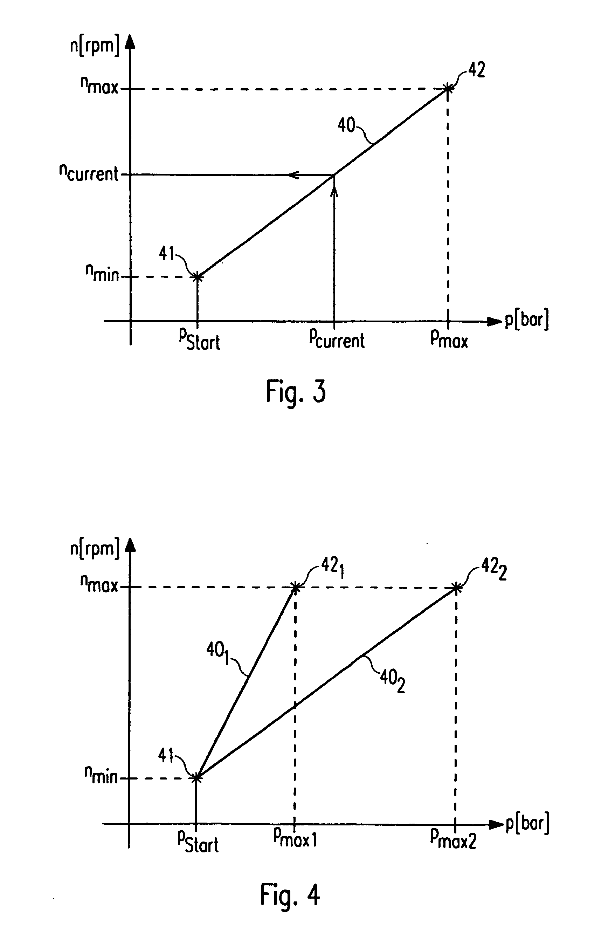

Die Vorgehensweise zur Umsetzung des Luftdrucks in ein Ansteuersignal ist schematisch in Fig. 3 dargestellt. Diese zeigt eine Kennlinie 40, gemäß der der Eingangsdruck in eine Drehzahl für den Motor umgesetzt wird. Diese Kennlinie 40 basiert auf zwei Bezugspunkten 41 und 42, welche im dargestellten Ausführungsbeispiel eine Gerade definieren. Ein Eingangs-Druckwert p_{Start} ist dabei einer Minimal-Drehzahl nₘᵢₙ zugeordnet. Erst bei Überschreiten dieses Eingangs-Druckwerts p_{Start} wird also der Elektromotor gestartet und mit der minimalen Drehzahl nₘᵢₙ betrieben. Bei Ansteigen des Drucks erfolgt dann linear auch eine Anhebung der Drehzahl, bis der Maximaldruck pₘₐₓ erreicht wird, der einer Maximal-Drehzahl nₘₐₓ für den Motor entspricht. Auf Basis dieser Kennlinie 40 kann also während des laufenden Betriebs einem aktuellen Druckwert p_{current} jederzeit eine entsprechende Drehzahl n_{current} zugeordnet werden. Eine Betätigung des Fußanlassers 5, über den die Druckluft der Einheit 1 variiert werden kann, führt also letztendlich zu einer Drehzahländerung des Elektromotors.

Anzumerken ist, dass die Kennlinie 40 nicht auf die dargestellte lineare Form beschränkt ist. Alternativ hierzu wäre es bspw. auch denkbar, zwischen den beiden Bezugspunkten 41 und 42 eine Kennlinie in Form einer Exponentialkurve einzusetzen, was dann die Möglichkeit eröffnen würde, im unteren Drehzahlbereich eine feinere Einstellung vorzunehmen.

Unabhängig von der Form der Kennlinie 40 ist allerdings für eine komfortable Steuerung des medizinischen Instruments 30 erforderlich, dass die Kennlinie 40 an die aktuellen Druckverhältnisse in optimaler Weise angepasst wird. Mit anderen Worten, der obere Bezugspunkt 42 sollte möglichst gut dem von dem System zur Verfügung gestellten Maximaldruck entsprechen, um die Steuermöglichkeiten voll ausschöpfen zu können. Da allerdings unterschiedliche zahnmedizinische Behandlungsplätze Druckluft in verschiedenen Druckbereichen zur Verfügung stellen, ist es erforderlich, für einen optimalen Betrieb der Vorrichtung 10 eine entsprechende Anpassung bzw. einen Abgleich durchzuführen. Die hierzu erfindungsgemäße Vorgehensweise ist schematisch in Fig. 4 dargestellt und soll nachfolgend erläutert werden.

Die erfindungsgemäße Lösung beruht darauf, dass nun nicht im Rahmen einer Initialisierungsprozedur ein Abgleich stattfindet. Stattdessen ist die Steuereinheit 20 der Vorrichtung 10 derart ausgeführt, dass sie während des laufenden Betriebs kontinuierlich den eingangsseitigen Luftdruck ermittelt und insbesondere auch den maximal gemessenen Druck speichert. Dieser Maximaldruck wird dann zur Bildung des oberen Bezugspunkts 42 herangezogen, während hingegen für den unteren Bezugspunkt 41 ein fest vorgegebener Eingangsdruckwert p_{Start} verwendet wird. Zwischen beiden Bezugspunkten wird dann wiederum - bspw. durch Interpolation - die Kennlinie 40 gebildet.

Fig. 4 zeigt hierbei zwei Fälle, wobei in einem ersten Fall das System einen maximalen Druck pₘₐₓ₁ zur Verfügung stellt. In diesem Fall wird dann automatisch die Kennlinie 40₁ mit dem zugehörigen oberen Endpunkt 42₁ gebildet. Für den Fall hingegen, dass im Laufe des Betriebs ein höherer Maximaldruck pₘₐₓ₂ ermittelt wird, wird der obere Bezugspunkt 42₂ automatisch angepasst und dementsprechend die Kennlinie 40₂ neu eingestellt.

Der Vorteil der erfindungsgemäßen Vorgehensweise besteht nunmehr darin, dass automatisch während des laufenden Betriebs eine für das entsprechende System optimal geeignete Kennlinie generiert wird. Letztendlich ist es ausreichend, wenn der Benutzer der Vorrichtung 10 beim erstmaligen Start mit Hilfe des Fußanlassers 5 eine maximale Leistung einstellt. Da dies üblicherweise standardmäßig durchgeführt wird, um kurz die Funktion des Systems zu überprüfen, ist also für diesen Abgleich kein zusätzlicher Aufwand erforderlich.

Der von der Steuereinheit 20 ermittelte maximale Druckwert pₘₐₓ wird ferner in dem Speicher 21 abgelegt, bei dem es sich vorzugsweise um einen nicht-flüchtigen Speicher handelt. Selbst nach einer vorübergehenden Deaktivierung der Vorrichtung 10 steht also bei einem Neustart automatisch die richtige Kennlinie zur Verfügung. Dabei besteht allerdings selbstverständlich die Möglichkeit, durch Betätigung des Tasters 16 die Einstellungen zu löschen, um einen neuen Abgleich durchführen zu können. Ferner kann in vorteilhafter Weise vorgesehen sein, dass der ermittelte maximale Druckwert bei jedem Neustart des Motors geringfügig herabgesetzt wird, um das System automatisch an leicht geänderte Druckbedingungen anzupassen.

Die zuvor beschriebene Vorgehensweise der Anpassung des oberen Bezugspunkts der Kennlinie auf Basis des über eine Spitzenwertdetektion ermittelten maximalen Drucks führt also zu einer einfachen und komfortablen Benutzung der gesamten Vorrichtung. Die Ergebnisse sind insbesondere dann hervorragend, wenn bei einer entsprechenden Betätigung des Fußpedals eine große Volumenänderung bzw. Druckänderung bewirkt wird. Bei Behandlungseinheiten allerdings, welche nur eine geringe Variation des Volumenstroms in der Treibluft hervorrufen, kann die Dynamik im resultierenden Druckbereich eingeschränkt sein, was möglicherweise zur Folge hat, dass nicht der volle Drehzahlbereich des Behandlungsinstruments ausgeschöpft werden kann.

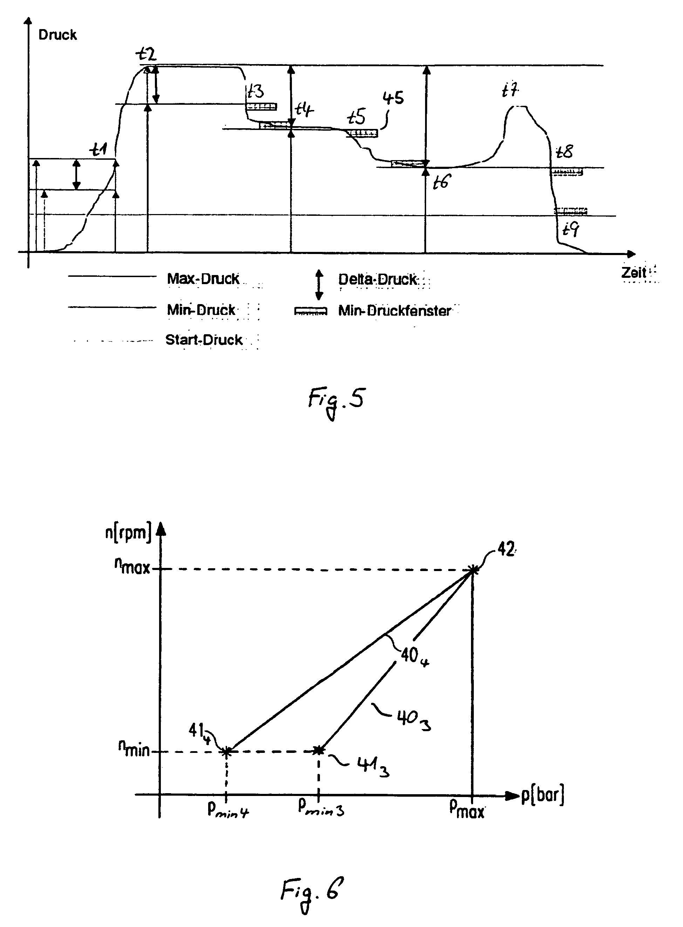

Um derartige Probleme zu vermeiden, ist in einer Weiterentwicklung der zuvor beschriebenen Vorgehensweise vorgesehen, dass auch der untere Bezugspunkt der Kennlinie automatisch angepasst wird. Dieser untere Bezugspunkt wird in diesem Fall also nicht mehr durch einen fest vorgegebenen Mindestdruck festgelegt, sondern basiert auf einem während des Betriebs ermittelten Minimaldruck. Diese Vorgehensweise soll nachfolgend anhand der Figuren 5 und 6 erläutert werden. Figur 5 zeigt hierbei beispielshaft den Verlauf der Druckkurve während eines Betriebs der Vorrichtung während hingegen Figur 6 die hieraus resultierende Anpassung der Kennlinie zeigt.

Ausgegangen wird hierbei von einem initialen Zustand zum Zeitpunkt t1. Dabei wird zunächst davon ausgegangen, dass in diesem initialen Zustand der Maximaldruck, der der maximalen Drehzahl entspricht, bei 1,8 bar liegt, während hingegen der Minimaldruck, der dem Startdruck und damit der Minimaldrehzahl entspricht, bei 1,0 bar liegt. Der zur Verfügung stehende Druckunterschied liegt dementsprechend zunächst bei 0,8 bar.

Nach dem Motorstart wird zunächst in der zuvor beschriebenen Vorgehensweise der Maximaldruck angepasst. Der gemessene Druckwert steigt entsprechend dem eingestellten Volumen und dem Systemdruck, wobei der Spitzenwert kontinuierlich gemessen und als aktueller Maximaldruck übernommen wird (Zeitpunkt t2). Hier wird zunächst der Minimaldruck unter Beibehaltung des initialen Deltadrucks von 0,8 bar ebenfalls angehoben.

Im weiteren Verlauf der Behandlung wird der Druck durch Verringerung der Betätigung am Pedal reduziert werden, was zur Folge hat, dass die Drehzahl bis zum Minimum abfällt. Da im dargestellten Ausführungsbeispiel allerdings der minimale Druck nicht stabil anliegt sondern stattdessen weiter abfällt, werden die Werte zunächst noch nicht angepasst (Zeitpunkt t3). Erst zum Zeitpunkt t4 liegt ein stabiler neuer Minimaldruck an. Hierfür ist Voraussetzung, dass der aktuelle Druck über eine bestimmte Fensterzeit von vorzugsweise 0,5 Sekunden im Wesentlichen stabil ist, also beispielsweise eine maximale Druckvarianz von 0,1 bar aufweist. Ferner liegt in diesem Fall der Druckwert unterhalb des alten Minimaldrucks, was zur Folge hat, dass der aktuelle Wert pₘᵢₙ₃ nun als neuer Minimaldruck angesehen wird. Da der Maximaldruck dem zum Zeitpunkt t2 ermittelten Wert entspricht, erfolgt also eine Anpassung des durch den Doppelpfeil dargestellten Druckbereichs, was zur Folge hat, dass der dynamische Bereich besser an den Pedal-Arbeitsbereich angepasst ist. Zu diesem Zeitpunkt t4 ergibt sich die in Figur 6 dargestellte Kennlinie 40₃ mit dem oberen Bezugspunkt 42 und dem unteren Bezugspunkt 41₃.

Entsprechend der in Figur 5 dargestellten Kurve ergibt sich aus einer weiteren Reduzierung der Pedalbetätigung eine neuerliche Druckreduzierung. Da der Druck in diesem Fall allerdings verhältnismäßig schnell fällt, wird das zum Zeitpunkt t5 dargestellte Druckfenster 45 nicht von links nach rechts überschritten. Der Druck wird dementsprechend nicht als ausreichend stabil angesehen, so dass keine neuerliche Anpassung des Minimaldrucks erfolgt. Erst zum Zeitpunkt t6 ist wiederum das zuvor beschriebene Kriterium erfüllt, d.h. der neuerlich abgefallene Druck wird als ausreichend stabil angesehen und der nunmehr vorliegende Wert pₘᵢₙ₄ wird als neuer Minimaldruck festgelegt, wodurch der dynamische Bereich (Deltadruck) weiter erhöht wird. Dementsprechend erfolgt eine neuerliche Anpassung der Kennlinie zu der neuen Kennlinie 40₄ mit dem neuen unteren Bezugswert 41₄.

Bei der sich anschließend in Figur 5 ergebenden Druckanhebung erfolgt keine Anpassung der Bezugswerte. Stattdessen wird die Drehzahl des Instruments entsprechend der aktuellen Kennlinie variiert. Anschließend wird bei dem dargestellten Beispiel der Druck durch Schließen des Pedals stark abgesenkt (Zeitpunkt t8). Hierbei wird die Solldrehzahl entsprechend dem aktuellen Druck bis zum Minimum gesenkt. Wird letztendlich zum Zeitpunkt t9 der Startdruck unterschritten, so wird der Motor vollständig gestoppt.

Aus der zuvor beschriebenen Vorgehensweise ergibt sich also, dass nunmehr auch der untere Bezugspunkt der Kennlinie angepasst werden kann, wobei ein neuer Minimaldruck immer dann bestimmt wird, wenn nach einem Druckabfall der Druck innerhalb des vorgegebenen Zeitraums im Wesentlichen konstant bleibt. Hierdurch ist sichergestellt, dass das Instrument über seinen vollen Drehzahlbereich hinweg angesteuert werden kann.

Letztendlich wird also durch die erfindungsgemäße Vorgehensweise die Benutzung der Vorrichtung deutlich vereinfacht. Insbesondere kann auf einen separaten Abgleich zur Inbetriebnahme der Vorrichtung verzichtet werden. Dabei ist nochmals darauf hinzuweisen, dass die ermittelten Eingangs-Druckluftwerte nicht zwingend in eine Drehzahl für den Motor umgesetzt werden müssen. Stattdessen wäre es auch denkbar, die Leistung, das Drehmoment und/oder die Abtragsleistung des von dem medizinischen Instrument betriebenen Werkzeugs einzustellen. Ferner könnte selbstverständlich von der Vorrichtung lediglich ein digitales Ansteuersignal ausgegeben werden, welches dann von dem Elektromotor in geeigneter Weise umgesetzt wird. Vorzugsweise übernimmt allerdings unmittelbar die Steuereinheit die vollständige Ansteuerung, indem dem Motor bereits ein der gewünschten Drehzahl bzw. Leistung entsprechender Versorgungsstrom übermittelt wird.

## Patentansprüche

1. Vorrichtung (10) zum Betreiben bzw. Ansteuern eines elektrisch betriebenen medizinischen Instruments (30), insbesondere eines zahnärztlichen Behandlungsinstruments, wobei das medizinische Instrument mit der Vorrichtung (10) zu verbinden ist,
und wobei die Vorrichtung (10) einen Anschluss (13) für eine Druckluftleitung (6) sowie eine Steuereinheit (20) aufweist, welche dazu ausgebildet ist, den Druck der zugeführten Druckluft entsprechend einer Kennlinie (40) in ein Ansteuersignal für das medizinische Instrument (30) umzusetzen,
**dadurch gekennzeichnet,**
**dass** die Steuereinheit (20) während des Betriebs kontinuierlich den maximalen Druck der Druckluft ermittelt und auf Basis dieses Maximaldrucks einen Bezugspunkt (42) der Kennlinie (40) bestimmt.

2. Vorrichtung nach Anspruch 1,
**dadurch gekennzeichnet,**
**dass** ein weiterer Bezugspunkt (41) der Kennlinie (40) einem vorgegebenen Mindestdruck entspricht.

3. Vorrichtung nach Anspruch 1,
**dadurch gekennzeichnet,**
**dass** ein weiterer Bezugspunkt (41) der Kennlinie (40) einem von der Steuereinheit (20) ermittelten Minimaldruck entspricht.

4. Vorrichtung nach Anspruch 3,
**dadurch gekennzeichnet,**
**dass** die Steuereinheit (20) während des Betriebs einen neuen Minimaldruck ermittelt, wenn nach einem Druckabfall unterhalb des aktuell festgelegten Minimaldrucks der Druck innerhalb eines vorgegebenen Zeitraums im Wesentlichen konstant bleibt, wobei vorzugsweise der Druck als im Wesentlichen konstant angesehen wird, wenn die Druckvarianz innerhalb von 0,5 Sekunden 0,1 bar nicht überschreitet.

5. Vorrichtung nach einem der Ansprüche 2 bis 4,
**dadurch gekennzeichnet,**
**dass** die Kennlinie (40) zwischen den beiden Bezugspunkten (41, 42) linear verläuft.

6. Vorrichtung nach einem der vorherigen Ansprüche,
**dadurch gekennzeichnet,**
**dass** diese einen nicht-flüchtigen Speicher (21) zur Speicherung des ermittelten Maximaldrucks sowie ggf. des ermittelten Minimaldrucks aufweist,
wobei vorzugsweise ein Eingabeelement (16) zum manuellen Löschen des gespeicherten Maximaldrucks sowie des auf. gespeicherten Minimaldrucks vorgesehen ist.

7. Vorrichtung nach Anspruch 6,
**dadurch gekennzeichnet,**
**dass** die Steuereinheit (20) bei jedem Startvorgang den gespeicherten Maximaldruck geringfügig reduziert sowie den ggf. gespeicherten Minimaldruck geringfügig anhebt.

8. Vorrichtung nach einem der vorherigen Ansprüche,
**dadurch gekennzeichnet,**
**dass** die Steuereinheit (20) entsprechend der Kennlinie (40) einen dem aktuellen Luftdruck entsprechenden Drehzahlwert für einen Elektromotor (32) des Instruments (30) ermittelt.

9. Vorrichtung nach einem der vorherigen Ansprüche,
**dadurch gekennzeichnet,**
**dass** die Vorrichtung (10) als Ansteuersignal für das medizinische Instrument (30) einen variablen Versorgungsstrom für einen Elektromotor (32) des Instruments (30) zur Verfügung stellt.

10. Verfahren zum Betreiben bzw. Ansteuern eines elektrisch betriebenen medizinischen Instruments (30), insbesondere eines zahnärztlichen Behandlungsinstruments,
wobei die Höhe des Drucks einer eingangsseitigen Druckluft entsprechend einer Kennlinie (40) in ein Ansteuersignal für das medizinische Instrument (30) umgesetzt und dem medizinischen Instrument (30) zugeführt wird,
**dadurch gekennzeichnet,**
**dass** während des Betriebs kontinuierlich der maximale Druck der Druckluft ermittelt und auf Basis dieses Maximaldrucks einen Bezugspunkt (42) der Kennlinie (40) bestimmt wird.

11. Verfahren nach Anspruch 10,
**dadurch gekennzeichnet,**
**dass** ein weiterer Bezugspunkt (41) der Kennlinie (40) einem vorgegebenen Mindestdruck entspricht.

12. Verfahren nach Anspruch 10,
**dadurch gekennzeichnet,**
**dass** ein weiterer Bezugspunkt (41) der Kennlinie (40) einem während des Betriebs ermittelten Minimaldruck entspricht.

13. Verfahren nach Anspruch 12,
**dadurch gekennzeichnet,**
**dass** während des Betriebs ein neuer Minimaldruck ermittelt wird, wenn nach einem Druckabfall unterhalb des aktuell festgelegten Minimaldrucks der Druck innerhalb eines vorgegebenen Zeitraums im Wesentlichen konstant bleibt,
wobei vorzugsweise der Druck als im Wesentlichen konstant angesehen wird, wenn die Druckvarianz innerhalb von 0,5 Sekunden 0,1 bar nicht überschreitet.

14. Verfahren nach einem der Ansprüche 10 bis 13,
**dadurch gekennzeichnet,**
**dass** die Kennlinie (40) zwischen den beiden Bezugspunkten (41, 42) linear verläuft.

15. Verfahren nach einem der Ansprüche 10 bis 14,
**dadurch gekennzeichnet,**
**dass** bei jedem Startvorgang der gespeicherte Maximaldruck geringfügig reduziert wird und ggf. der gespeicherte Minimaldruck geringfügig angehoben wird.

16. Verfahren nach einem der Ansprüche 10 bis 15,
**dadurch gekennzeichnet,**
**dass** entsprechend der Kennlinie (40) ein dem aktuellen Luftdruck entsprechender Drehzahlwert für einen Elektromotor (32) des Instruments (30) bestimmt wird.

17. Verfahren nach einem der Ansprüche 10 bis 16,
**dadurch gekennzeichnet,**
**dass** als Ansteuersignal für das medizinische Instrument (30) ein variabler Versorgungsstrom für einen Elektromotor (32) des Instruments (30) zur Verfügung gestellt wird.

## Claims

1. A device (10) for operating or controlling an electrically operated medical instrument (30), in particular a dental treatment instrument, wherein the medical instrument is to be connected to the device (10),
and wherein the device (10) has a connection (13) for a compressed-air line (6) and also a control unit (20) which is constituted to convert the pressure of the compressed air that is delivered, in accordance with a characteristic line (40), into a control signal for the medical instrument (30),
**characterised in that**
during operation the control unit (20) continuously ascertains the maximum pressure of the compressed air and on the basis of this maximum pressure determines a reference point (42) of the characteristic line (40).

2. A device according to claim 1,
**characterised in that**
a further reference point (41) of the characteristic line (40) corresponds to a predetermined minimum pressure.

3. A device according to claim 1,
**characterised in that**
a further reference point (41) of the characteristic line (40) corresponds to a minimal pressure ascertained by the control unit (20).

4. A device according to claim 3,
**characterised in that**
the control unit (20) ascertains a new minimal pressure during operation if after a drop in pressure below the currently established minimal pressure the pressure remains substantially constant within a predetermined period of time,
wherein the pressure is preferably considered to be substantially constant if the pressure variance does not exceed 0.1 bar within 0.5 seconds.

5. A device according to any of claims 2 to 4,
**characterised in that**
the characteristic line (40) runs linearly between the two reference points (41, 42).

6. A device according to any preceding claim,
**characterised in that**
it has a non-volatile memory (21) for the storage of the maximum pressure ascertained and also, if applicable, the minimal pressure ascertained,
wherein preferably an input element (16) is provided for manually deleting the stored maximum pressure and also the, if applicable, stored minimal pressure.

7. A device according to claim 6,
**characterised in that**
with each start process the control unit (20) reduces the stored maximum pressure slightly and also raises the, if applicable, stored minimal pressure slightly.

8. A device according to any preceding claim,
**characterised in that**
the control unit (20), in accordance with the characteristic line (40), ascertains for an electric motor (32) of the instrument (30) a rotational-speed value that corresponds to the current air pressure.

9. A device according to any preceding claim,
**characterised in that**
the device (10) provides, as a control signal for the medical instrument (30), a variable supply current for an electric motor (32) of the instrument (30).

10. A method for operating or controlling an electrically operated medical instrument (30), in particular a dental treatment instrument,
wherein the level of the pressure of compressed air on the input side is converted, in accordance with a characteristic line (40), into a control signal for the medical instrument (30) and delivered to the medical instrument (30),
**characterised in that**
during operation the maximum pressure of the compressed air is ascertained continuously and on the basis of this maximum pressure a reference point (42) of the characteristic line (40) is determined.

11. A method according to claim 10,
**characterised in that**
a further reference point (41) of the characteristic line (40) corresponds to a predetermined minimum pressure.

12. A method according to claim 10,
**characterised in that**
a further reference point (41) of the characteristic line (40) corresponds to a minimal pressure ascertained during operation.

13. A method according to claim 12,
**characterised in that**
a new minimal pressure is ascertained during operation if after a drop in pressure below the currently established minimal pressure the pressure remains substantially constant within a predetermined period of time,
wherein the pressure is preferably considered to be substantially constant if the pressure variance does not exceed 0.1 bar within 0.5 seconds.

14. A method according to any of claims 10 to 13,
**characterised in that**
the characteristic line (40) runs linearly between the two reference points (41, 42).

15. A method according to any of claims 10 to 14,
**characterised in that**
with each start process the stored maximum pressure is reduced slightly and, if applicable, the stored minimal pressure is raised slightly.

16. A method according to any of claims 10 to 15,
**characterised in that**
in accordance with the characteristic line (40) a rotational-speed value for an electric motor (32) of the instrument (30) corresponding to the current air pressure is determined.

17. A method according to any of claims 10 to 16,
**characterised in that**
as a control signal for the medical instrument (30) there is provided a variable supply current for an electric motor (32) of the instrument (30).

## Revendications

1. Dispositif (1) pour le fonctionnement ou l'amorçage d'un instrument (30) médical à fonctionnement électrique, en particulier un instrument de soin dentaire, sachant que l'instrument médical est à relier au dispositif (10),
et sachant que le dispositif (10) présente un raccord (13) pour un conduit d'air comprimé (6) ainsi qu'une unité de commande (20), qui est réalisée pour convertir la pression de l'air comprimé amené, conformément à une courbe caractéristique (40), en un signal d'amorçage pour l'instrument (30) médical,
**caractérisé en ce**
**que** l'unité de commande (20) calcule, au cours du fonctionnement en continu, la pression maximale de l'air comprimé et détermine, sur la base de ladite pression maximale, un point de référence (42) de la courbe caractéristique (40).

2. Dispositif selon la revendication 1,
**caractérisé en ce**
**qu'**un point de référence (41) supplémentaire de la courbe caractéristique (40) correspond à une pression minimale prédéfinie.

3. Dispositif selon la revendication 1,
**caractérisé en ce**
**qu'**un point de référence (41) supplémentaire de la courbe caractéristique (40) correspond à une pression minimale déterminée par l'unité de commande (20).

4. Dispositif selon la revendication 3,
**caractérisé en ce**
**que** l'unité de commande (20) calcule, au cours du fonctionnement, une nouvelle pression minimale lorsque la pression reste essentiellement constante pour une durée prédéfinie après une chute de pression sous la pression minimale fixée à l'instant t, sachant que de préférence la pression est réputée essentiellement constante lorsque l'écart de pression ne dépasse pas en l'espace de 0,5 seconde 0,1 bar.

5. Dispositif selon l'une quelconque des revendications 2 à 4,
**caractérisé en ce**
**que** la courbe caractéristique (40) est linéaire entre les deux points de référence (41, 42).

6. Dispositif selon l'une quelconque des revendications précédentes,
**caractérisé en ce**
**que** ledit dispositif présente une mémoire (21) non volatile servant à mémoriser la pression maximale calculée ainsi qu'éventuellement la pression minimale calculée, sachant que de préférence un élément de saisie (16) servant à supprimer manuellement la pression maximale mémorisée ainsi que la pression minimale éventuellement mémorisée est prévu.

7. Dispositif selon la revendication 6,
**caractérisé en ce**
**que** l'unité de commande (20) réduit légèrement, lors de chaque démarrage, la pression maximale mémorisée et relève légèrement la pression minimale éventuellement mémorisée.

8. Dispositif selon l'une quelconque des revendications précédentes,
**caractérisé en ce**
**que** l'unité de commande (20) calcule, conformément à la courbe caractéristique (40), une valeur de vitesse de rotation correspondant à la pression de l'air à l'instant t pour un moteur électrique (32) de l'instrument (30).

9. Dispositif selon l'une quelconque des revendications précédentes,
**caractérisé en ce**
**que** le dispositif (10) fournit, en tant que signal d'amorçage pour l'instrument (30) médical, un courant d'alimentation variable pour un moteur électrique (32) de l'instrument (30).

10. Procédé servant à faire fonctionner ou à amorcer un instrument (30) médical à fonctionnement électrique, en particulier un instrument de soin dentaire,
sachant que l'intensité de la pression d'un air comprimé côté entrée est convertie, conformément à une courbe caractéristique (40), en un signal d'amorçage pour l'instrument (30) médical et est amenée à l'instrument (30) médical,
**caractérisé en ce**
**que** la pression maximale de l'air comprimé est calculée lors du fonctionnement en continu, et en ce qu'un point de référence (42) de la courbe caractéristique (40) est déterminé sur la base de ladite pression maximale.

11. Procédé selon la revendication 10,
**caractérisé en ce**
**qu'**un point de référence (41) supplémentaire de la courbe caractéristique (40) correspond à une pression minimale prédéfinie.

12. Procédé selon la revendication 10,
**caractérisé en ce**
**qu'**un point de référence (41) supplémentaire de la courbe caractéristique (40) correspond à une pression minimale calculée lors du fonctionnement.

13. Procédé selon la revendication 12,
**caractérisé en ce**
**qu'**une nouvelle pression minimale est calculée lors du fonctionnement lorsque la pression reste essentiellement constante pour une durée prédéfinie après une chute de pression sous la pression minimale fixée à l'instant t,
sachant que de préférence la pression est réputée essentiellement constante lorsque l'écart de pression ne dépasse pas en l'espace de 0,5 seconde 0,1 bar.

14. Procédé selon l'une quelconque des revendications 10 à 13,
**caractérisé en ce**
**que** la courbe caractéristique (40) est linéaire entre les deux points de référence (41, 42).

15. Procédé selon l'une quelconque des revendications 10 à 14,
**caractérisé en ce**
**que** lors de chaque démarrage, la pression maximale mémorisée est légèrement réduite, et en ce qu'éventuellement la pression minimale mémorisée est légèrement relevée.

16. Procédé selon l'une quelconque des revendications 10 à 15,
**caractérisé en ce**
**qu'**une valeur de vitesse de rotation correspondant à la pression de l'air à l'instant t est déterminée pour un moteur électrique (32) de l'instrument (30) conformément à la courbe caractéristique (40).

17. Procédé selon l'une quelconque des revendications 10 à 16,
**caractérisé en ce**
**qu'**un courant d'alimentation variable est fourni pour un moteur électrique (32) de l'instrument (30) en tant que signal d'amorçage pour l'instrument (30) médical.
